# EUROPEAN PATENT APPLICATION

(11) **EP 4 732 807 A1**
(43) Date of publication of application: **29.04.2026**
(21) Application number: 24209067.8
(22) Date of filing: 25.10.2024
(51) Int. Cl.: A61B 90/20

(54) **SYSTEMS AND METHODS FOR MIXED-REALITY SUPPORTED 3D NAVIGATION FOR MUSCULOSKELETAL SURGERY BASED ON X-RAY IMAGES WHILE THE PATIENT IS MOVING**

(71) Applicant: metamorphosis GmbH, 33100 Paderborn (DE)
(72) Inventor: KRAUS, Florian, 83075 Bad Feilnbach (DE); REIMER, Stefan, 33100 Paderborn (DE)
(74) Representative: LKGLOBAL Partnerschaftsgesellschaft mbB

(57) **Abstract**

A computer implemented method for determining a 3D position of a surgical object relative to a region of interest in a patient's anatomy is suggested. The method comprising the steps of receiving an X-ray image and a 3D data set. The X-ray image is a 2D projection image generated with an imaging direction and defining a 3D image coordinate system. When applying the method, the 2D projection image may depict a part of a patient's anatomy and at least a part of the surgical object. The received 3D data set describes the part of the patient's anatomy and a region of interest in a 3D data coordinate system. In order to determine a spatial relation between the 3D image coordinate system and the 3D data coordinate system, the part of the patient's anatomy is localized based on the X-ray image. Subsequently, the 3D position of the surgical object relative to the region of interest is determined in the 3D data coordinate system based on (I) the determined spatial relation between the first 3D image coordinate system and the 3D data coordinate system, (II) a spatial relation between the surgical object and the first 3D image coordinate system, and on (III) information related to the geometrical aspect.

## Description

### FIELD OF INVENTION

The invention relates to the fields of computer- and robot-assisted surgery. Further, the invention relates to systems and methods providing information related to surgical objects and anatomy based on CT data, or another type of 3D X-ray scan, and X-ray images. In particular, the invention relates to systems and methods for automatically determining spatial position and orientation of a surgical object relative to a patient while the patient is moving. The methods may be implemented as a computer program executable on a processing unit of the systems.

### BACKGROUND OF THE INVENTION

Spinal surgery, and in particular, spinal fusion, has become a commonly performed surgical procedure. However, surgery on the spine also poses substantial risks to the patient. An incorrect drilling performed on the spine may damage the spinal cord, which can cause serious injuries to the nerves or the covering of the spinal cord, potentially leading to chronic pain, permanent paralysis, incontinence, or sexual dysfunction. In order to mitigate these risks due to incorrect drillings, computer-assisted navigation is already used with some frequency in spinal surgery. Computer assistance concerns navigating the surgeon, ensuring that drillings are performed in the correct location, pedicle screws are properly placed, and so on. This entails determining the precise relative 3D position between a surgical tool (such as a drill) and the patient (e.g., relative to a desired drilling trajectory).

Almost all existing navigation systems require additional procedural steps and apparatuses like 3D cameras, trackers, reference bodies, and so on. In navigated spinal surgery, most current systems use optical tracking, where dynamic reference frames are attached to the spine (for tracking of any patient movement) and a reference body is attached to the surgical tool. All references must then be at all times visible to a 3D camera. Such an approach has multiple disadvantages, including but not limited to:
- Plausibility and accuracy of the registration must be continuously watched. Registration may have to be repeated in case of tracker movements.
- If a tracker movement goes unnoticed, navigation instructions will be incorrect, possibly resulting in harm to the patient.
- Accuracy decreases with increasing distance from the camera.

In summary, existing navigation systems are error-prone. The long chain of errors in the navigation workflow of registration, instrument calibration and real time tracking means that navigation systems can only be as accurate as the underlying weakest part of the chain. Therefore, even when working with navigation systems, surgeons need to constantly ensure the accuracy of the navigation instructions, e.g. by tactile and landmark checking, which is an uncertain validation procedure.

Robot-assisted surgery systems are becoming more popular due to the precision they are thought to provide. However, as robots work substantially on the instructions provided by navigation systems, robots can only be as accurate as the information provided by those navigation systems. Because existing navigation systems are error-prone, current robotic surgeons cannot be trusted to autonomously perform any surgical procedure. Rather, robotic surgeons are simple automated holding arms that hold an instrument. The actual drilling still needs to be performed by a surgeon.

The main problem with conventional navigation systems is that they do not determine the actual relative 3D positions between surgical tools and anatomy but rather infer those positions and orientations by tracking, with a camera, reference bodies externally affixed to tools (e.g., a drill) and anatomy. This camera, by its nature, can only see the externally fixated reference body but not the drill itself inside the bone. If either one of the reference bodies moves, or if the drill bends inside the bone, the navigation system will be oblivious to this fact and provide incorrect information, possibly resulting in harm to the patient.

It is desirable to have a navigation system that (i) does not require any additional procedures and apparatuses for navigation, and (ii) is capable of reliably determining the actual 3D positions and orientations of surgical objects (e.g., tools or implants) relative to a patient. This shall also be robustly possible after or during a movement of patient or patients anatomy, e.g. navigating while operating on thoracic spine, which is moving due to patient's breathing or due to other patient's movements, e.g. a movement due to applied pressure, which can e.g. occur during a drilling procedure like distal locking of a long nail in a long bone or inserting a screw like a pedicle screw or pelvic screw.

While such a navigation system may be particularly useful for spinal surgery, it may also be used for many other surgical procedures where the spatial relation of a surgical object or implant relative to the patient must be precisely determined. It may even be used in combination with the mentioned conventional navigation system so as to provide redundant navigation information and, thus, to make the navigation highly robust and this level of safety may enable autonomous robotic surgery.

### SUMMARY OF THE INVENTION

At least the one or the other of the mentioned problems are mitigated or solved by the subject matter according to each of the independent claims. Further embodiments are described in the respective dependent claims.

Systems and methods are proposed, which require neither reference bodies nor trackers, to determine the 3D spatial relation or 3D position of a surgical object relative to a part of a patient's anatomy.

A spatial relation may be understood as constraining parameterization with at least one degree of freedom to partially or fully describe the position and orientation between two 3D coordinate systems (e.g. (1) the reference system of a surgical object and (2) the 3D coordinate system of an X-ray image). Correspondingly, the position and orientation between an object and a coordinate system may be partially or fully described.

For example, 2, 3, 4 or 5 degrees of freedom of a surgical object may be determined in an image 3D coordinate system. Based only on a single 2D projection image, the spatial position of an object in an image 3D coordinate system may e.g. be determined by 5 degrees of freedom. In the context of this disclosure, this is denoted as "localization", meaning the position in the X-ray direction is significantly less accurately determined than its 2D position in the image and it's additional 3 degrees of freedom (DOF) of rotation. Further, some components of the 3D orientation might also show reduced accuracy, or are entirely indetectable, but still be localized. A cylindric object may be described via two accurate positional degrees of freedom and one accurate rotational degree of freedom, wherein the other degrees of freedom are less accurate or even undetermined.

Throughout this disclosure, the term "imaging direction" (also called "viewing direction") onto an object (or region of interest) means the 3D angle at which an X-ray beam passes through a chosen point of the object (or region of interest). For example, the imaging direction onto the region of interest describes the orientation of the X-ray machine relative to the anatomy within the region of interest. The imaging direction may be determined as part of the localization of an object (e.g. patient anatomy), which emphasizes that this is a spatial relation between the object (e.g. region of interest) and the X-ray image 3D coordinate system.

Based on a single projection image, it may be the case that the object is too thin to reliably determine its tilt rotation in direction of the image depth, due to the facts that it is not completely visible in the image (which typically is always true for a drill bit), its imaging depth cannot be accurately determined. In this case, the determination of 4DOF may be sufficient as an intermediate step in order to determine 5DOF or even 6DOF based on an additional step like receiving information about the 3D position of an anchor point in a coordinate system like the 3D coordinate system of another object of interest like a patient's anatomy. This may allow to determine its 3D position and also its tilt relative to the other obj ect.

It may be the case that determining 3DOF in translational position and 2DOF in rotational position is completely sufficient for a navigation task. This may especially be true for determining the relative 3D position of, e.g., a drill bit relative to a patient's anatomy. In this case determining the rotational position around the drill axis may not be of relevance.

6DOF sufficiently describe the position and rotation for any static object. Some objects, like Kirchner-Wire or Drill-Bits, show deformations, like bending, thus cannot be fully described by 6DOF, but could only be described with more degrees of freedom, e.g. 8DOF. For cases like this, it still may be sufficient to focus on a subset of degrees, which sufficiently describe the parts of the object that are of interest. For instance, describing the tip position of the bent drill-bit with 3DOF, or localizing the region around the tip with 4DOF.

The proposed systems and methods (implemented as computer program product) are configured to determine a 3D spatial relation based on an X-ray image depicting at least one surgical object (at least partially) and part of the patient's anatomy in order to determine 3D positions of the at least one surgical object relative to the patient's anatomy at one point in time.

Further, a real time tracking of tools (without the need of any additional trackers or references attached to the patient's anatomy) may be provided in order to determine a 3D position of at least one surgical object relative to patient's anatomy at another point in time. This may be the case even if the patient's anatomy (the region of interest) is moving.

In general, a system for determining a 3D position of a surgical object relative to a region of interest in a patient's anatomy is suggested. The system comprises a processing unit and a computer program product including instructions executable on the processing unit. By means of the computer program product, a computer implemented method can be performed, the method comprising the steps of receiving an X-ray image and a 3D data set. The X-ray image is a 2D projection image generated with an imaging direction and defining a 3D image coordinate system. When applying the method, the 2D projection image may depict a part of a patient's anatomy and at least a part of the surgical object. The received 3D data set describes the part of the patient's anatomy and a region of interest in a 3D data coordinate system.

In order to determine a spatial relation between the 3D image coordinate system and the 3D data coordinate system, the part of the patient's anatomy is localized based on the X-ray image.

Subsequently, the 3D position of the surgical object relative to the region of interest is determined in the 3D data coordinate system based on (I) the determined spatial relation between the first 3D image coordinate system and the 3D data coordinate system, (II) a spatial relation between the surgical object and the first 3D image coordinate system, and on (III) information related to the geometrical aspect.

The spatial relation between the surgical object and the 3D image coordinate system is derived by one or more of (A) inferring a neural network, (B) utilizing a plurality of virtually generated x-ray images, with the images depicting different parts of the surgical object from different imaging directions, and (C) applying an algorithm utilizing a 3D model representing at least a part of the surgical object.

The information related to the geometrical aspect includes one or more of (a) the geometrical aspect of the surgical object is positioned near a surface of at least part of the region of interest, (b) a point, an axis or a plane in the first 3D image coordinate system which constraints the possible options for spatial relation of the geometrical aspect, (c) a 3D position of a target point in the 3D data coordinate system, which is near to the geometrical aspect, with the projections in the 2D projection image of the target point and the projection of the geometrical aspect in the 2D projection image being near to each other.

Aspects of the disclosure may be applied in open, minimally invasive, endoscopic and percutaneous spinal surgery in degenerative, deformity, tumor, trauma or infection treatments, including but not limited to cervical and thoracolumbar discectomies, decompression procedures, motion preserving surgery, stabilization procedures such as vertebroplasty and kyphoplasty, fusion procedures such as ACDF, TLIF, PLIF, LLIF, OLIF, XLIF, open, minimally invasive or percutaneous pedicle screws, tumour debulking or corpectomy procedures.

The surgical object may be a surgical instrument or tool like, e.g., a drill, a needle, or a chisel, or it may be an implant like, e.g., a pedicle screw, a nail, or a plate. The surgical object has a known geometric shape, which is described by the 3D model. The 3D model can be a detailed abstraction of the object, e.g. describing its surface and density, or less detailed, e.g. containing length, diameter, or partial surface descriptions.

The surgical object may also have a point denoted as "anchor point", whose position may be known in the 3D model of the surgical object, and which can be identified in the X-ray image. The anchor point may for instance be the tip of a drill. In case of a chisel, the anchor point may be one edge or the midpoint of the chisel's blade. The anchor point could be more abstract, like the center of a cylindric surgical tool, which is not necessarily part of the object, or an axis going through the center of the object.

The 3D data set describing part of the patient's anatomy may be generated by a computerized tomography (CT) scan or some other type of 3D X-ray scan, which may be obtained pre- or intra-operatively. The 3D data set includes the region of interest, which must be sufficiently rigid. The region of interest may be, e.g., a volume containing a bone fragment, a particular vertebra, or two neighboring vertebrae provided that these may be assumed to be sufficiently rigid. The region of interest may also be the entire 3D data set. Because the 3D data set accurately describes the anatomy of the patient at least within the region of interest, a one-to-one correspondence between points in the physical world and points in the 3D data set may be established at least within the region of interest. The 3D data set may thus define a 3D coordinate system in physical 3D space. It may be an aim to determine the surgical object's 3D position in this 3D coordinate system or, in other words, to determine the surgical object's 3D position (in the physical world) relative to the region of interest within the patient's anatomy.

According to an embodiment, in pre- or intra-operative planning, a target point in the 3D data set and within at the 3D data set of the patient's anatomy is determined. The target point may be determined pre- or intraoperatively, either manually by the surgeon, automatically by the system, or semi-automatically by the surgeon with some automated support by the system. Hence, the target point's position in the 3D coordinate system defined by the 3D data set is known. The target point may be chosen such that the anchor point of the surgical object may be placed on (or close to) the physical location of the target point at the patient, which may be achieved, e.g., by choosing the target point on a bone surface.

In another embodiment, the target point may be the start point of a target path, whose end point will be called the target end point. The target path lies within the region of interest, and its position and orientation within the 3D data set may be determined in pre- or intra-operative planning, either manually by the surgeon, automatically by the system, or semi-automatically by the surgeon with some automated support by the system. Therefore, if a target path is available, its position and orientation in the 3D coordinate system is known. For instance, when treating a vertebra, the target path may correspond to the planned drilling trajectory (typically a straight line), and the target point is located on the surface of the vertebra.

The methods disclosed herein teach how to determine the 3D position of at least one surgical object in the 3D coordinate system defined by the 3D data set that describes the patient's anatomy. By doing that, the relative 3D position of the surgical object relative to the region of interest of the patient's anatomy is determined. Therefore, if the region of interest includes a planned target path, the relative 3D position of the surgical object relative to the target path in the patient's anatomy may also be determined. It may also be an object to provide instructions in order to align the surgical object with the target path (if available), e.g., in order to drill along the target path.

It is noted that some surgical objects, such as a drill bit, have rotational symmetry around an axis. For such objects, it suffices to determine five degrees of freedom of their 3D position relative to the patient's anatomy because the rotation around the object's axis is irrelevant. For instance, in case of a drill bit, it suffices to determine the 3D position of the drill tip and the 3D orientation of the drill axis.

In the context of this disclosure, the rotation of the surgical object around its axis may be irrelevant for the 3D spatial relation of the surgical object relative to anatomy. In other words, the 3D position is deemed as determined irrespective of whether or not the actual rotational angle of the surgical object about its rotation axis is exactly determined.

Neither the target point nor the target path is necessarily required. According to an embodiment, it is also possible to work with an anchor point (e.g., the tip of a drill - being a geometrical anchor aspect) only. In this case, the surgeon may decide intra-operatively whether the 3D position and orientation of the surgical object relative to the patient's anatomy is satisfactory, and, if not, how it should be corrected.

It is emphasized that the 3D data set need not necessarily be segmented. In particular, it is not necessary to identify bone surfaces in the 3D data set (other than choosing the target point on a bone surface). Furthermore, the precise location and shape of the region of interest within the 3D data set need not be known and need not be explicitly determined. For instance, when treating a vertebra, a CT scan may contain this vertebra and also some neighboring vertebrae (and also surrounding soft tissue). Initially, the vertebra need not be identified in the 3D data set. A rough approximation of the region of interest within the 3D data set may be derived from the target point. The anchor point defines a 2D region of interest within the X-ray image. For subsequent computations, data points within the 2D region of interest may be weighted, where points further away from the anchor point receive a lower weighting.

The localization of the 3D region of interest depicted in the X-ray image may be determined by computing, from the 3D data set, digitally reconstructed radiographs (DRRs) for a multitude of localizations. Alternatively, a model of the imaged structure may be utilized for determining the localization. The localization may also be determined based on position sensors and/or movement sensor provided in the imaging device. The localization could also be determined by tracking the imaging device with any tracking device, for instance a mixed reality (XR) device, starting from a previous registration of the 3D data and the 3D image coordinate system, and adding the detected relative movement of the imaging device onto the previous localization of the previous registration.

When taking a DRR best matching the X-ray image to determine the localization, it may also be possible to use a numerical optimizer to find an optimal DRR. It is not necessary to restrict the DRR to the region of interest. When assessing the best match between DRR and X-ray, the region of interest may be emphasized (e.g., with an appropriate weighting). If the X-ray image acquired suffers from distortion (i.e., it was acquired with an image intensifier rather than with a flat-panel receiver), it may be necessary to use a smaller region in the X-ray image for the DRR match (or to emphasize points close to the anchor point) because the larger the distance between points, the stronger the distortion may be. Because distortion typically affects areas further away from the central X-ray beam more strongly, it may be helpful to ensure that the anchor point lies close to the center of the X-ray image.

As discussed in detail in US2021/0248779 A1, the 3D position of a thin surgical object such as a drill bit cannot always be uniquely determined based on a single X-ray image. Therefore, in general there may be an ambiguity in the relative 3D spatial relation between the surgical object and the patient's anatomy if only one X-ray image is available. The present disclosure teaches to resolve such an ambiguity by first determining the 3D position of the anchor point in the 3D coordinate system defined by the 3D data set. Different methods are disclosed how this can be achieved.

A method according to an embodiment, which does not use a target point, requires the anchor point to be located on a bone surface. Based on a single X-ray image, the 2D position of the anchor point is determined in the X-ray image, and the imaging direction of the X-ray image onto the region of interest may be determined from a DRR match. In 3D space, the points whose virtual projections into the X-ray image lie on the anchor point determine a line. Because the anchor point which is a geometrical anchor aspect, lies on the bone surface, the position of the anchor point in 3D can be found by determining the point on the line that lies on the bone surface. It is sufficient to determine the location of the bone surface along the line. A segmentation of the entire bone's surface is not necessary. It is understood that "at" or "on the bone surface" also includes that it is sufficient that the anchor point is in the vicinity of the bone surface, e.g. less than 2.5mm or less than 1.5mm away from the bone surface. Alternatively, it may be precisely known which distance to the bone surface the anchor aspect has. In this case this distance can be taken into account.

Another method establishes a correspondence between the anchor point (on the surgical object) and the target point (which may be a geometrical aspect of the 3D data set). This is done by virtually projecting the target point into the X-ray image, which utilizes the known imaging direction onto the region of interest determined through, e.g., a DRR match.

If the location of the anchor point matches the location of the target point in the X-ray image, it may be assumed that the 3D position of the anchor point matches the 3D position of the target point in physical space. This is because the target point is chosen such that the anchor point of the surgical tool may be placed (in physical space) on the target point. Of the three coordinates of the 3D position, two degrees of freedom are determined by the X-ray image, and the third degree of freedom is determined by the a priori information that the target point and the anchor point lie on the bone surface or at a defined distance from it.

In case the anchor point is not on the bone surface because e.g. drilling a hole in a pedicle has already started, yet another method to determine the 3D position of the anchor point in the 3D coordinate system defined by the 3D data set uses a further X-ray image from a different imaging direction. This further X-ray image may be acquired at a previous point in time, concurrently with the first X-ray image at the first point in time (e.g., if a G-arm is used), or at a later point time (e.g., if a robot is used that can hold the surgical object still). The further X-ray image depicts at least part of the surgical object and the region of interest within the patient's anatomy. The 2D position of the anchor point in the further X-ray image is determined. The imaging direction onto the region of interest of the further X-ray image is determined, which allows registering the further X-ray image with the first X-ray image. This method assumes that there was no movement of the anchor point relative to the anatomy in between the generation of the two X-ray images. An advantage of this method is the fact that no preoperative planning (e.g., defining drilling trajectories and/or target points) is needed. Moreover, if the anchor point (e.g., the tip of a drill) lies below the bone surface, the likelihood of an undesired slipping of the surgical tool is minimized.

An anchor point may also be a geometrical aspect of the surgical tool, like the tip and central axis of a drill bit. Estimating the 3D position can be achieved when the anchor point can be partially localized in the X-ray image in the first point in time or partially localized in the further point in time. For example, the central axis of a drill bit might be identified in the X-ray image in the first point in time, and in the X-ray image of the further point in time only the tip of the drill bit could be identified. Under the assumption that the drill tip was not moved, it is still sufficient to estimate the 3D position of the anchor point in the 3D coordinate system: Given the imaging directions onto the drill bit of the X-rays in both the first and the further point in time, the detected tip and central axis in the images lead to geometrical aspects that can be transformed from the image 3D coordinate systems to the 3D coordinate system. The tip of the drill bit in the further point in time describes a line in the 3D coordinate system, and the central axis of the first point in time describes a plane in the 3D coordinate system. The intersection of the line and the plane provide the estimate of the 3D position of the anchor point. The 3D orientation of the central axis of the anchor point is only partially estimated in this case, since a more precise estimation would require identifying the central axis in both X-ray images.

Estimating the 3D position of an anchor aspect which may be a geometrical aspect may also be achieved in a similar manner, even when the surgical tool was moved. In the example of a drill bit, the anchor aspect of the surgical tool may be its central axis direction and its tip position. The drill bit may be placed in a 3D coordinate system of the anatomy, e.g. within a bone. A first X-ray may be acquired in a first point in time, in which the drill bit may be localized such that its tip was determined in 2D coordinates (at least 2DOF). The first X-ray image corresponds to a 3D image coordinate system, in which the localization of the tip corresponds to an uncertainty line between the X-ray source and the projected tip in the 2D image plane. At a second point in time, another X-ray may be acquired, where also a registration between the 3D image coordinate systems and the 3D coordinate system of the anatomy was received or determined.

At the second point in time, the drill bit might have been inserted further in a way, that the tip is not on the same 3D position (relative to the anatomy coordinate system) compared to the first point in time, but the tip in the first point in time is still near the axis of the drill bit in the second point in time. The drill bit may be localized in the second X-ray image, such that its tip and axis are determined in 2D (at least 3DOF), where the tip corresponds to an uncertainty line in the second 3D image coordinate system and the axis corresponds to a plane in the second 3D coordinate system.

Using the registration between the coordinate systems, the uncertainty line from the first 3D image coordinate system, as well as the uncertainty line and plane from the second 3D image coordinate system may be transformed into the 3D coordinate system of the anatomy. In this 3D coordinate system, the intersection of the uncertainty plane of the second X-ray image and the uncertainty line from the first X-ray is a 3D point in the axis of the drill bit in the second point in time.

To fully determine the 3D position of the drill tip and drill axis, another degree of freedom needs to be constrained, which could have several origins. Examples are, (i) the angle may have been determined the drill bit from the first X-ray (i.e. at least 3DOF), e.g. when the direction remained the same between the first and second X-ray; (ii) the localization of the drill bit in the second X-ray may provide a more detailed estimate about the rotation (i.e. at least 4DOF), (iii) a reasonable assumption about a fourth degree of freedom may exist. With this constraint, the axis of the drill bit in the second point in time may be determined. Given the axis of the drill bit, the tip point of the drill bit in the second point in time may be determined as the intersection between the axis of the drill bit and its uncertainty line from the second X-ray image. Given the tip position and the axis direction of the drill bit (i.e. 3D position of the anchor aspect), 5DOF are determined for the second point in time. The rotation of the drill bit around its main may also be determined as part of the localization in the second X-ray image.

Geometrical aspects of objects are descriptive attributes. Examples of geometrical aspects are a subset of the surface of a drill bit, a characterizing point at the tip, a centroid of the surface of a bone, a projected trajectory of a drill bit, a bent central axis of a bent cylindric surgical tool. Depending on the geometrical aspect, fewer than 6DOF may be required to fully describe it in relation to a 3D coordinate system. A tip of the drill bit may be fully described by its position with 3DOF, a line requires only 5DOF. In the case of a bent cylindric surgery tool, the central axis may have more than 6DOF to fully describe it, but it may be sufficient to partly estimate its properties with reduced set of parameters.

Typically, the 3D data set (e.g., the CT scan) contains scaling information (i.e., information about sizes) for the anatomy described in the data set. Moreover, a scale of the scene depicted in the X-ray may also be provided by the depicted surgical object whose exact geometric shape is known, or other surgical objects of known geometry that may be depicted in the X-ray image (e.g., an already implanted pedicle screw). This redundancy with respect to scale information may be used to differentiate between different surgical objects in the X-ray image if these are of identical geometric shape but with different sizes (e.g., drills with different diameters), by comparing the scale of the 3D data and the size of the surgical object. If the X-ray image acquired suffers from distortion, the same information (i.e., the 3D data set, the 3D model of the surgical object, or another surgical object of known geometry) may also be used to correct the distortion.

If the location of the anchor point does not match the location of the (virtually projected) target point in the X-ray image, instructions may be provided by the system how to move the surgical tool such that the anchor point is closer to the target point, after which a new X-ray may be acquired. If the location of the anchor point is in the vicinity of the target point in the X-ray image and a local model of the bone surface in the vicinity of the target point is available, the 3D position of the anchor point may be obtained, for the 2D position of the anchor point detected in the X-ray image, from the local model. This suffices to determine the 3D relative spatial relation between the surgical object and the patient's anatomy. Such a local model of the bone surface may, for instance, be a median shape of the bone, a first-order approximation of the shape of the bone, or a local segmentation of the bone surface in the vicinity of the target point. The correct model may be selected based on a labeling of the 3D data set (e.g., classifying the vertebra), which may be done automatically, semi-automatically, or manually. The size of the vicinity of the target point may be adapted based on the local model, e.g., the less variation the local model has close to the target point, the larger the vicinity may be chosen. Of course, in case that a segmentation of the 3D data set (i.e., a model of the entire bone surface) is available, this can be taken into account, which may enhance accuracy.

It may be the case that the 3D position of the anchor aspect has large error in some dimensions, while being sufficiently accurate in the dimensions to adjust, for example when navigating a surgical tool to a target point. When navigating the anchor aspect to the target point, the navigational information may be constrained to a translation in 2D (e.g. surface of a bone), while the 3^{rd} dimension of the translation may only be estimated with larger error. This is especially helpful, when the 2D navigation may be estimated based on an X-ray image, where the 2D navigation directions are not perpendicular to the projection plane of the X-ray image.

Alternatively or additionally, the localization of the anchor aspect may utilize a target point to constrain the 3D coordinates. For example, the drill tip could be estimated in 2D in the X-ray image, while the position on the uncertainty line (i.e. line from the X-ray source to the projected anchor aspect in the image plane) could be assumed to be close to the target point, given that the target point is estimated or known in the 3D image coordinate system.

Alternatively or additionally, the system may include means to track the anchor aspect from the time of the acquired X-ray to any later point in time, utilizing the estimated position of the anchor aspect in the 3D image coordinate system relative to the target point. The tracking may be done via external sensors, tracking via camera or from the instructions or sensors of a surgical robot. Based on this tracking, the direction and distance to the target point may be updated and provided to a surgeon or robot in real time (or near real time).

It will be understood, that the target point needs to be linked to the estimate of the anchor aspect to provide a navigational instruction. For example, the target point may be derived from a 3D data set, where the 3D coordinate system of the 3D data set was registered with the 3D image coordinate system, such that the target point can be mapped to the 3D image coordinate system.

As mentioned above, the precise location and shape of the 3D region of interest within the 3D data set need not be known. Moreover, it is not necessary to establish a precise correspondence between the 2D region of interest in the X-ray image and the 3D region of interest in the 3D data set. There may, however, be cases where there are several possible 3D regions of interest. For instance, in spinal surgery, there may be a plurality of 3D regions of interest because a plurality of vertebrae is to be treated. In such a case, a human surgeon may identify manually which 3D region of interest is to be treated. It may also be possible to automatically select the relevant 3D region of interest, corresponding to the anchor point indicated with the surgical tool, by performing a DRR match for each possible 3D region of interest and selecting the best such match.

The region of interest is an approximation of the area (or volume) that can be assumed to be sufficiently rigid so that the 3D data set describes the patient's anatomy within the region of interest with sufficient accuracy. For example, since a spine is flexible, individual vertebrae may move relative to each other. If the patient was moved after acquiring the 3D data set, some relative movement of individual vertebrae would have to be expected. This means that the area that may be assumed to be rigid may be larger with an intra-operative 3D X-ray (without subsequent patient movement) than with a pre-operative CT scan (with subsequent patient movement), assuming no significant movement due to breathing.

The 3D data set may be input to the system or may be derived by the system. For example, the system could use a statistical model of relevant parts of the anatomy to estimate 3D data based on an X-ray. As an example, in context of spinal surgery, a statistical model (e.g. active shape model) may describe the surface or density of one or more vertebra. The 3D data set may also be updated or extended in further X-ray images for improved accuracy and reliability.

To improve the accuracy of the derived 3D data set, the system may utilize a geometrical aspect of a surgical object by registering the aspect between two or more images. For example, a drill bit may be placed on a bone in a first and a second X-ray image, where the drill axis might change between the images, but the tip stays the same. Similarly, a screw might be identified in both X-ray images in order to constrain the possible viewing directions of the X-ray images. This may be used to determine or constrain the registration of the 3D image coordinate systems, thus improving its accuracy.

Alternatively or additionally, a priori knowledge about the geometrical aspect of a surgical tool may be utilized. For example, if a drill tip is placed on the surface of a bone, then the modelled bone surface needs to be close to the detected drill tip position. Using the localization of the drill tip may constrain the system in estimating the 3D data, thus improving the accuracy of the estimation.

The methods taught in this disclosure may also complement existing navigation technologies. Another aspect may be a continual incorporation of information from intraoperative X-rays. While the systems and methods disclosed herein do not require a camera or other sensor for navigation, it may nevertheless (for increased accuracy and/or redundancy) be combined with a camera or other sensor (e.g., a sensor attached to the robot) for navigation. Information from the X-ray image and information from the camera or other sensor may be combined to improve the accuracy of the determination of relative spatial position and orientation, or to resolve any remaining ambiguities (which may, for instance, be due to occlusion). If the tool is held by a robot or robotic arm, information provided by the robot or robotic arm itself (e.g., about the movements it has performed) may also be considered.

It is also possible to combine the methods taught in this disclosure with an extended reality device such as head-mounted augmented or mixed reality glasses. For instance, by tracking a power tool with a head-mounted extended reality device, the change of 3D position and 3D orientation of a drill bit inserted into the power tool between two points in time may be determined. Hence, if the 3D position of a surgical object (such as a drill bit) relative to anatomy is known at a first point in time, then by tracking the power tool with an extended reality device, the 3D position of the surgical object relative to anatomy can be determined at a second point in time. If it is known that only the tilt of a surgical object was changed between the two points in time (e.g., the power tool was only tilted, but the drill tip remains in place), then this knowledge may be used by the system.

It is noted that the information about change of 3D position of a surgical object between two points in time may also be provided by a robotic arm holding the surgical object because robotic arms are typically capable of tracking their movements in 3D space.

Possible indications for applying this disclosure include any type of bone drilling, e.g., for insertion of a screw into a pedicle, wedge osteotomy, a screw into a sacroiliac joint, a screw connecting two vertebrae, a screw through the pelvis, a screw through the acetabulum, or a drilling for crucial ligament. The disclosed teaching may be used, e.g., for drilling, reaming, milling, chiseling, sawing, resecting, and implant positioning, hence it may also support, e.g., osteotomy, laminectomy, tumor resection, total hip replacement.

It may be understood that the system may comprise a processing unit and that the method may be implemented as computer program product which can be executed on that processing unit.

According to an embodiment, the system and method may cause an imaging device to generate an X-ray image or some type of 3D X-ray scan (e.g., a CT scan). Additionally or alternatively, the system and method may control the imaging device to move to a new position for generating an X-ray image from a different imaging direction. Such different imaging direction may be the appropriate imaging direction as suggested by the system. The current imaging direction may be determined on the basis of internal movement and/or position sensors of the imaging device.

It is noted that the image data of the processed X-ray image may be received directly from an imaging device, for example from a C-arm, G-arm, or biplanar 2D X-ray device, or alternatively from a database. A biplanar 2D X-ray device has two X-ray sources and receivers that are offset by any angle.

According to an embodiment, objects in the X-ray image may automatically be classified and identified in an X-ray projection image. However, an object may also be manually classified and/or identified in the X-ray projection image. Such a classification or identification may be supported by the device by automatically referring to structures that were recognized by the device.

It is noted that a processing unit may be realized by only one processor performing all the steps of the process, or by a group or a plurality of processors, which need not be located at the same place. For example, cloud computing allows a processor to be placed anywhere. For example, a processing unit may be divided into a first sub-processor that controls interactions with the user, including a monitor for visualizing results, and a second sub-processor (possibly located elsewhere) that performs all computations. The first sub-processor or another sub-processor may also control movements of, for example, a C-arm or a G-arm of an X-ray imaging device.

According to an embodiment, the device may further comprise storage means providing a database for storing, for example, X-ray images. It will be understood that such storage means may also be provided in a network to which the system may be connected, and that data related to a neural net may be received over that network. Furthermore, the device may comprise an imaging unit for generating at least one 2D X-ray image, wherein the imaging unit may be capable of generating images from different directions.

According to an embodiment, the system may comprise a device for providing information to a user, wherein the information includes at least one piece of information out of the group consisting of X-ray images and instructions regarding step of a procedure. It will be understood that such a device may be a monitor or an augmented reality device for visualization of the information, or it may be a loudspeaker for providing the information acoustically. The device may further comprise input means for manually determining or selecting a position or part of an anatomical structure in the X-ray image, such as a bone outline, for example for measuring a distance in the image. Such input means may be for example a computer keyboard, a computer mouse, or a touch screen, to control a pointing device like a cursor on a monitor screen, which may be included in the device. The device may also comprise a camera or a scanner to read the labeling of a packaging or otherwise identify a surgical object. A camera may also enable the user to communicate with the device visually by gestures or mimics, e.g., by virtually touching devices displayed by virtual reality. The device may also comprise a microphone and/or loudspeaker and communicate with the user acoustically.

According to an embodiment, the system may also comprise an extended reality device. It will be understood that extended reality devices cover all devices on the extended reality spectrum and are therefore a superset of virtual reality, augmented reality and mixed reality devices. This represents all devices which can be worn by a user and can (1) alter the perception of the user via any sense - including vision, hearing, olfaction, gustation and tactile perception - and (2) can track its environment via a variety of sensor information - including cameras, depth sensors, accelerometers, and magnetometers. Those sensors may track the environment including an imaging device and/or at least one object like an anatomy model, a surgical tool, or an implant. It is noted that the tracking information allows for a differentiation of objects and/or devices. For example, the extended reality device may be head-mounted and comprise a pair of glasses through which the user may see the environment, but which are configured to visualize information or a virtual representation of an object in the field of view of the user.

It is noted that all references to C-arm movements in this disclosure always refer to a relative repositioning between C-arm and patient. Hence, any C-arm translation or rotation may in general be replaced by a corresponding translation or rotation of the patient/OR table, or a combination of C-arm translation/rotation and patient/table translation/rotation. This may be particularly relevant when dealing with extremities since in practice moving the patient's extremities may be easier than moving the C-arm. It is noted that the required patient movements are generally different from the C-arm movements, in particular, typically no translation of the patient is necessary if the target structure is already at the desired position in the X-ray image. The system may compute C-arm adjustments and/or patient adjustments. It is furthermore noted that all references to a C-arm may analogously apply to a G-arm, O-arm, or similar.

The methods and techniques disclosed herein may be used in a system that supports a human user or surgeon, or they may also be used in a system where some or all of the steps are performed by a robot. Hence, references to a "user" or "surgeon" in this patent disclosure may refer to a human user as well as a robotic surgeon, a mechanical support device, or a similar apparatus. Similarly, whenever it is mentioned that instructions are given how to adjust the C-arm, it is understood that such adjustments may also be performed without human intervention, i.e., automatically, by a robotic C-arm, by a robotic table, or they may be performed by OR staff with some automatic support. It is noted that because a robotic surgeon and/or a robotic C-arm may operate with higher accuracy than humans, iterative procedures may require fewer iterations, and more complicated instructions (e.g., combining multiple iteration steps) may be executed. A key difference between a robotic and a human surgeon is the fact that a robot may keep a tool perfectly still in between acquisition of two X-ray images.

A computer program product may preferably be loaded into the random-access memory of a data processor. The data processor or processing unit of a system according to an embodiment may thus be equipped to carry out at least a part of the described process. Further, the disclosure may relate to a computer-readable medium such as a CD-ROM on which the disclosed computer program product may be stored. However, the computer program product may also be presented over a network like the World Wide Web and can be downloaded into the random-access memory of the data processor from such a network. Furthermore, the computer program product may also be executed on a cloud-based processor, with results presented over the network.

It is noted that prior information about a surgical object (e.g., the size and type of a drill bit) may be obtained by simply scanning of a packaging (e.g., the barcode) or any writing on the surgical object itself, before or during surgery.

As should be clear from the above description, a main aspect is a processing of X-ray image data, allowing an automatic interpretation of visible objects. The methods described herein are to be understood as methods assisting in a surgical treatment of a patient. Consequently, the method may not include any step of treatment of an animal or human body by surgery, in accordance with an embodiment.

It will be understood that steps of methods described herein, and in particular of methods described in connection with workflows according to embodiments some of which are visualized in the figures, are major steps, wherein these major steps might be differentiated or divided into several sub-steps. Furthermore, additional sub-steps might be between these major steps. It will also be understood that only part of the whole method may constitute the invention, i.e. steps may be omitted or summarized.

It has to be noted that embodiments are described with reference to different subject-matters. In particular, some embodiments are described with reference to method-type claims (computer program product) wherein other embodiments are described with reference to apparatus-type claims (system/device). However, a person skilled in the art will gather from the above and the following description that, unless otherwise specified, any combination of features belonging to one type of subject-matter as well as any combination between features relating to different subject-matters is considered to be disclosed with this application.

The aspects defined above and further aspects, features and advantages of the present invention can also be derived from the examples of the embodiments to be described hereinafter and are explained with reference to examples of embodiments also shown in the figures, but to which the invention is not limited.

The tip of an object like the tip of a sleeve may or may not be depicted in the 2D X-ray image as it might be occluded by other parts of the same object or by other objects. Still it may be possible to correctly detect enough of the outline and potentially also use inner appearance of the sleeve in the X-ray image and thus use this information to determine its relative 3D position and 3D orientation relative to the anatomy. This information may then be used as basis for the tracking of the object e.g. by cameras and/or 3D sensors of a head mounted display. By tracking the 3D position of a sleeve a drilling trajectory may be determined and/or observed although the drill or drilling machine itself may not be tracked at all. This may be an advantage as tracking a drilling machine in order to determine the actual drilling trajectory may result in inaccuracies or even significant deviation between the drilling trajectory and the trajectory of the drilling machine e.g. because the part of the drillbit outside of the bone (and outside of a sleeve or in absence of a sleeve) may significantly bend which is especially true for long drill bits.

It may be an advantage to combine the tracking (i.e. the determination of a spatial relation t additional points in time, e.g. real-time tracking) of a sleeve in order to determine a drilling trajectory and the tracking of a drilling machine in order to determine the drilling depth of a drill bit in e.g. a bone.

It may be an advantage to track a relative position of the drilling machine relative to the sleeve. This tracking may be sufficient if done only in one dimension, e.g. only the distance of the (calculated) position of the drill tip relative to the position of the sleeve tip in direction of the drilling trajectory.

In order to ensure a robust tracking of a sleeve, the sleeve may be shaped in a way that enough parts of it are not occluded by other objects like, instruments, drilling machine or hand or arm of a surgeon.

In order to ensure a robust detection and determination of the 3D position of a sleeve e.g. relative to an anatomy, based on of the on the image processing of the 2D X-ray image, it may be an advantage to have enough radio opaque features of the sleeve or its rigidly attached sleeve holding arm that spans enough volume.

It may be an advantage that the part of the sleeve actually covering parts of the drill bit may be translucent in order to enable a robust detection and image processing of enough part of the drill bit.

After incision the procedure may start with positioning/navigating a drill sleeve. As soon as the drill sleeve is positioned on the surface of the anatomy at the desired/planned entry point a drill bit attached to an insertion tool (e.g. a power tool) may be inserted in the drill sleeve until it touches the bone surface. A real time tracking system (e.g. a head mounted augmented reality device or a robotic arm) may track the insertion tool and detects when it is on the surface. This detection may be made more robust by e.g. rotating the insertion tool as soon as the tip of the drill bit has reached the surface. As the drill sleeve may always stay with its anchor point on the surface of the anatomy, it will move together with a patient's breathing movements. Any change in angulation of the drill sleeve may be determined by a tracking system that may be provided e.g. by a head mounted display and its cameras and/or 3D sensory devices. When the drilling starts, tracking of the insertion tool and the sleeve may allow to update a navigational information about the 3D position of the drill bit inside the bone/patient's anatomy.

Summarizing, a device may be provided for measuring a drilling depth in a moving anatomy. The device may comprise a tracking system for tracking surgical tools and a processing unit for processing information received from the tracking system, wherein the surgical tools comprise at least a drill sleeve and a drill bit at a drilling machine, wherein the tracking system tracks the 3D position of the drill sleeve, with the drill sleeve moving together with the anatomy. Further, the tracking system tracks the 3D position of the drill bit, with the drill bit is moving within the drill sleeve and relative to the anatomy.

Based on tracked 3D positions of the drill sleeve and on tracked 3D positions of the drill bit at a plurality of points in time, a processing unit of the device may be configured to determine a 3D position of the drill sleeve relative to the 3D position of the drill bit at each of the plurality of points in time and may be configured to consequently determine a maximum distance of a distal tip of the drill bit from the drill sleeve.

The maximum distance of the drill tip from the drill sleeve may correspond to the maximum drilling depth of the drill bit into a bone, when the drill sleeve is contact with an outer surface of the bone.

### BRIEF DESCRIPTION OF THE DRAWINGS

Fig. 1 shows a 2D X-ray projection image depicting a spine from anterior-posterior direction.
Fig. 2 shows a slice of a 3D scan of the same spine as Fig. 1, depicting a sagittal cross-section.
Fig. 3 shows a slice of the same 3D scan as Fig. 2, depicting an axial cross-section.
Fig. 4 shows an example workflow for performing a pedicle screw drilling based on planning.
Fig. 5 shows an example short workflow for performing a pedicle screw drilling without changing imaging direction but knowledge about the bone surface.
Fig. 6 shows an example workflow for performing a pedicle screw drilling based on a further X-ray image with different imaging direction.

Throughout the drawings, the same reference numerals and characters, unless otherwise stated, are used to denote like features, elements, components, or portions of the illustrated embodiments. Moreover, while the present disclosure will now be described in detail with reference to the figures, it is done so in connection with the illustrative embodiments and is not limited by the particular embodiments illustrated in the figures.

### DETAILED DESCRIPTION OF THE EMBODIMENTS

The present disclosure teaches systems and methods how to determine the 3D position and orientation of a surgical object relative to a part of a patient's anatomy, called the region of interest, which is described by a 3D data set such as a CT scan or some other type of 3D X-ray scan, based on a single X-ray image. Without additional information, the 3D pose (i.e., 3D position and orientation) of a thin surgical object such as a drill cannot always be uniquely determined based on a single X-ray image.

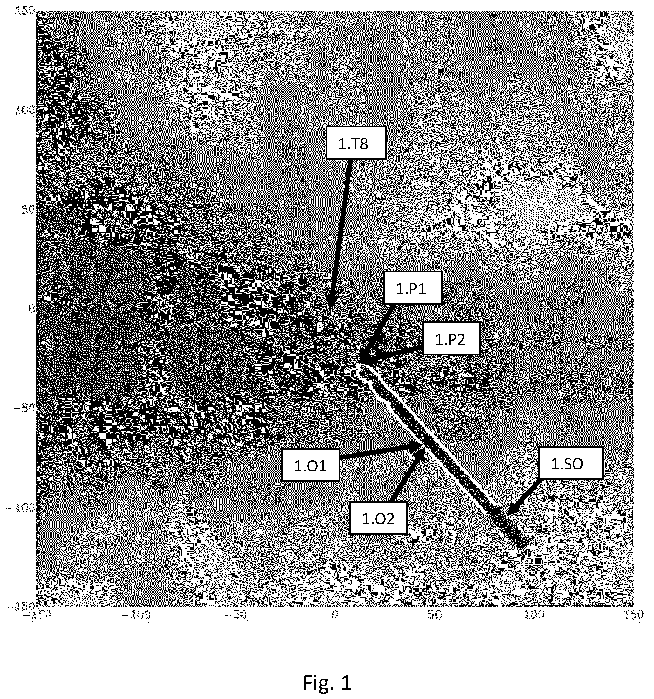

Fig. 1 shows a 2D X-ray projection image depicting a spine from anterior-posterior direction. A surgical object (1.SO) is placed such that its tip lies on the bone surface of vertebra T8 (1.T8). Two outlines of the surgical object are shown for two different 3D positions (1.P1 and 1.P2, respectively) and 3D orientations of the surgical object for which the outlines (the white solid lines marked as 1.O1 and 1.02, respectively) are more or less identical. The tip of the surgical object in 3D space lies on the line defined by the drill tip and the X-ray source (typically a focal point) of the X-ray machine, i.e., the epipolar line of the tip of the surgical object. Depending on the 3D position of the tip of the surgical object, the 3D orientation may be adjusted such that the projected outline of the surgical object fits its appearance in the 2D X-ray image. It is emphasized that there is an infinite number of combinations of 3D positions and corresponding 3D orientations leading to a nearly identical outline of the surgical object, of which two are depicted in Fig. 1.

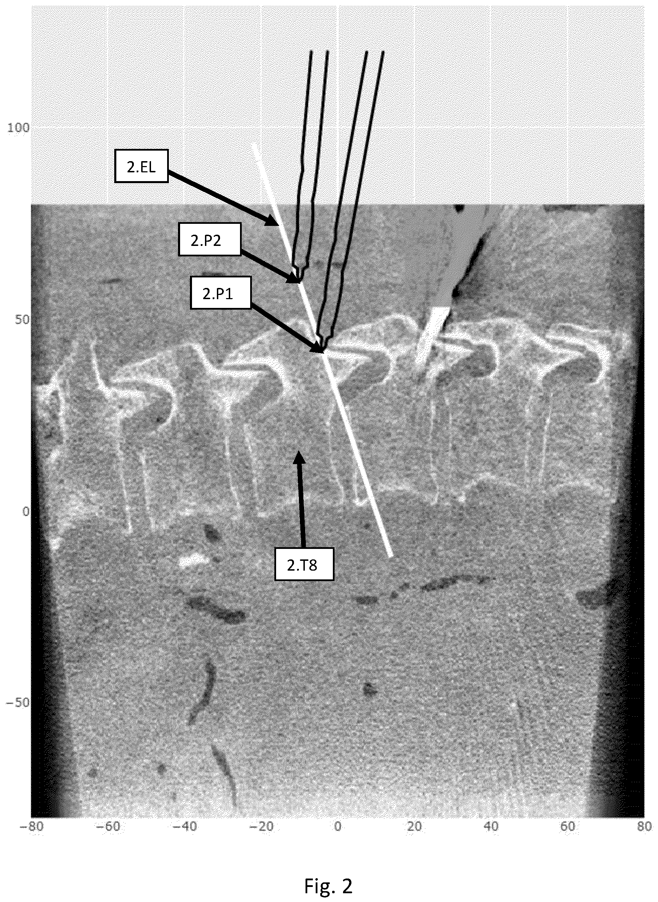

Fig. 2 shows a slice of a 3D scan of the same spine as Fig. 1, depicting a sagittal cross-section. A white line (2.EL) shows all possible 3D positions of the tip of the surgical object, corresponding to the epipolar line of the tip of the surgical object from Fig. 1. Two points on the line are chosen, one (2.P1) such that the tip of the surgical object lies on the bone surface of vertebra T8 (2.T8), and the other (2.P2) at a certain distance from the bone surface. Depending on the 3D position of the surgical object it is clearly visible that the 3D orientations of the surgical object differ, but both leading to nearly identical outlines in the X-ray projection image in Fig. 1.

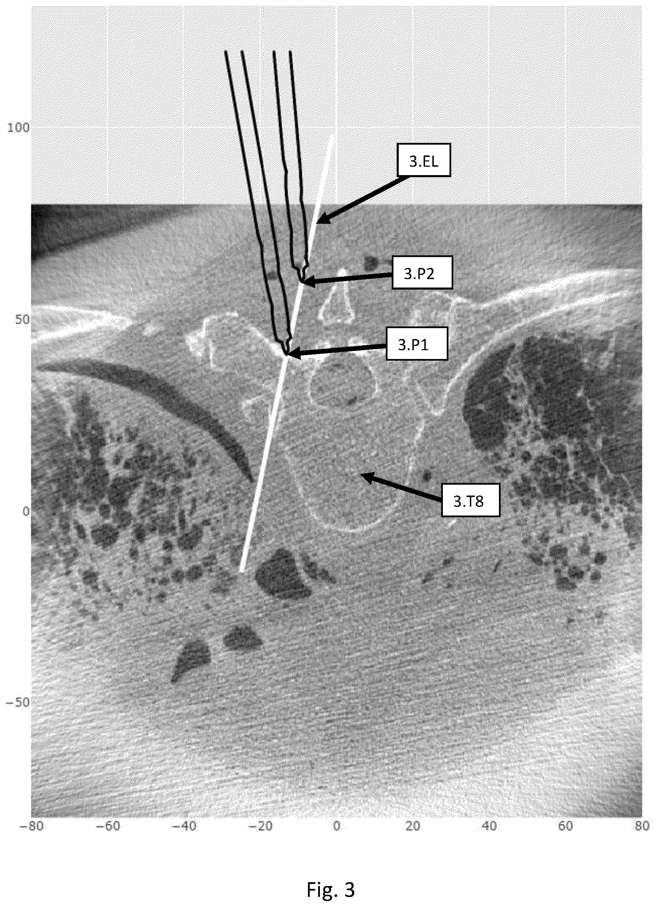

Fig. 3 shows a slice of the same 3D scan as Fig. 2, depicting an axial cross-section. A white line (3.EL) shows all possible 3D positions of the tip of the surgical object, corresponding to the line 2.EL from Fig. 2. As in Fig. 2, two points on the line are chosen, one (3.P1) such that the tip of the surgical object lies on the bone surface of vertebra T8 (3.T8), and the other (3.P2) at a certain distance from the bone surface. Again, it is visible that the 3D orientations of the surgical object differ, but both leading to nearly identical outlines in the X-ray projection image in Fig. 1.

In other words, Figures 1 to 3 show two different 3D constellations with two different drill poses, which lead to an identical (or nearly identical) X-ray projection image. The two drill constellations differ in the imaging depth (which is the distance from the image plane, i.e., from the X-ray receiver) and tilt (i.e., 3D orientation). However, if it is possible to remove the ambiguity regarding imaging depth (due to some a priori information), the drill pose relative to the patient's anatomy may be uniquely determined.

The present disclosure teaches to remove the ambiguity regarding imaging depth by establishing a correspondence between the anchor point of the surgical object and the target point (whose position in the 3D coordinate system defined by the 3D data set is known), utilizing the a priori information that the target point and the anchor point lie on the bone surface or at a defined distance from it. This may be done based on a single X-ray image.

Alternatively, the ambiguity regarding imaging depth may also be removed without using any target point. According to an embodiment, the tip of a drill (i.e., the anchor point) is placed on a bone surface near the intended drilling path (i.e., within the region of interest), and an X-ray image is acquired. The drill tip's position is detected in the X-ray image. For the imaging direction onto the region of interest as determined based on a DRR match of the 3D data set to the X-ray image, the possible drill tip positions in 3D space whose virtual projections into the X-ray image lie on the detected anchor point determine a line (a so-called epipolar line) in the 3D coordinate system defined by the 3D data set. Because of the a priori knowledge that the drill tip lies on the bone surface, the position of the drill tip in the coordinate system defined by the 3D data set can be found by determining the point on the line that intersects (i.e, lies on) the bone surface. Finding the bone surface along the line may be done automatically, e.g., by evaluating the Hounsfield values in the 3D data set along the line (or in a vicinity around the line) and searching for strong gradients. Of course, if a local segmentation of the bone surface in the relevant region is already available, this may be used for intersecting the line.

It may be possible to increase the accuracy of determining the spatial relation between the surgical object and the region of interest by acquiring further X-ray images from the current and/or different imaging directions.

It is also possible to determine the 3D spatial relation of the surgical object relative to the region of interest after the surgical object has been inserted into the patient, i.e., the anchor point is no longer located on the bone surface. This is of interest, for instance, when performing a drilling. In such a case, at a first point in time, an X-ray image is acquired, from which the drilling start point in the coordinate system of the 3D data is determined, which is assumed to be the 3D position of the drill tip (i.e., the anchor point) at the first point in time. After advancing the tool into the patient, at a second point in time, a further X-ray image is acquired. The ambiguity regarding the 3D drill pose may now be resolved based on the assumption that the drill axis, as determined at the second point in time, runs through the drilling start point as determined at the first point in time. This assumption requires that the drilling actually started at the anchor point as determined at the first point in time. This assumption may be particularly easy to justify if a surgical robot is used because this eliminates unintentional movements of the surgical object, and movements of the patient may be detected by the single-image registration. Patient movement may also be detected in real time by a head-mounted augmented reality device (e.g., using a camera integrated into the augmented reality device). If, at any point in time, the system detects patient movement, it may request a new X-ray image, or it may request that the procedure be repeated, or it may use the detected movement of the anatomy to improve the estimate of the drill pose relative to the anatomy.

In case the patient movement is large enough relative to the expected movement of the drill bit, simply detecting the drill bit movement (without tracking an independent object) may be sufficient to detect, that the patient was moved. This may be used to warn the surgeon, e.g. estimating that the drill might have slipped from the desired position.

Similarly, if the breathing movement is large enough compared to the drill tip movement, it may even suffice to identify it as component of the drill tip movement, and model it as a periodic movement that can be subtracted of the drill tip position in order to improve the accuracy of the 3D position of the drill tip relative to the moving anatomy.

An alternative and likely more accurate approach to track the patient movement is to track a second object to allow for improved accuracy of drill tracking relative to the anatomy in the region of interest. For example, a system may track the patient directly (e.g. the visible skin or tissue around the position of the entry of the drill bit); an indirect example is to estimate the position of the sleeve independently of the drill bit, where the sleeve can be assumed to remain on the bone surface during drilling that mainly allows to detect the drilling depth relative to the moving bone surface. Overall, tracking the anatomical area of interest directly or indirectly allows to reduce the noise in tracking the drill position relative to the area of interest, improving accuracy of surgery steps (e.g. in terms of drill guidance for a surgeon or a surgical robot).

It is understood, that tracking an object like the drill, the drilling machine, the sleeve, or the patient anatomy may be done in real time, or at arbitrary points in time. It may be the case, that the tracking is not available during the entire time, e.g. due to occlusion, due to placement of objects which lead to unacceptable ambiguities, or due to voluntary interruptions of the tracking process.

To utilize the tracking of the drill, it is beneficial to link it to the 3D coordinate system of the 3D data or the 3D coordinate systems of the X-ray images. For this, an object visible in the X-ray needs to be tracked outside of it, to estimate a spatial relation between the tracking system and the other 3D coordinate systems. This may require that the systems are somewhat synchronized, so a tracked drill can be mapped to a detected drill in a specific X-ray. It may also be sufficient to estimate the orientation and placement of the patient based on a priori information (including calibration steps) to generate a sufficiently accurate spatial relation. The registration of the coordinate systems is not the focus of the system disclosed herein and may be approached in a different manner.

This procedure may be repeated, possibly also acquiring X-ray images from other imaging directions, e.g., for increased accuracy.

If the system directly controls an imaging device, new images may be acquired automatically. If the system controls a robotic imaging device, it may automatically acquire an image from a desired imaging direction.

According to an embodiment, such a system may be particularly useful for spinal surgery. The following three workflows are examples how this system may be used to perform a pedicle screw drilling for spinal fusion.

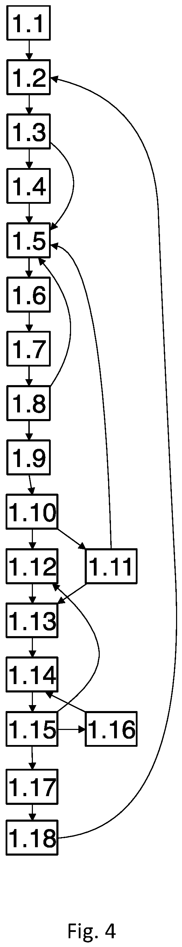

### Example workflow for performing a pedicle screw drilling with planning (cf. Fig. 4)

It is noted that the numbering of the steps in the workflow of Fig. 4 is a combination of a "1." indicating that the workflow is the first one out of three, followed by an actual number of the step as used in the following description of the workflow.
1. The surgeon and/or the system performs a pre-operative planning in the 3D data set (e.g., a CT scan or 3D X-ray scan) by determining a target path, i.e., the planned drilling trajectory, within the 3D data set, where the target point is the starting point of the intended drilling trajectory, which is located on the bone surface of the pedicle, and the target end point is the end point of the intended drilling trajectory.
2. The surgeon positions the drill tip on the dorsal bone surface in approximate vicinity of the pedicle to be drilled in (in case the relative position of drill and bone is already known, the drill tip may be positioned more precisely).
3. If the system utilizes external real time (or near real time) tracking, it may continuously track the drill bit, the drilling machine or the sleeve, to at least partially determine the drill position independently of the X-rays.
   If a tracking system is not available, it may continue at Step 5.
4. The system may continuously detect a second reference (e.g. anatomy in region of interest or tracking of the sleeve) and/or model the movement of the region of interest to improve the tracking accuracy of the drill bit relative to the region of interest.
5. The surgeon acquires an X-ray image (e. g., approximately in anterior-posterior (AP) imaging direction), preferably holding the drilling machine in such a way that the surgeon's hand is not in the X-ray beam. If an imaging direction were to lead to the surgeon's hand being in the X-ray beam, the system may provide instructions for a different imaging direction.
6. The system detects the position of the drill tip (i.e., the anchor point) in the X-ray image. The system localizes the region of interest in the X-ray image by computing, from the 3D data set, digitally reconstructed radiographs (DRRs) for a multitude of possible localizations. The DRR best matching localization is selected. It is not necessary to restrict the DRR to the region of interest. The system may use a weighting function to match the important regions within the 2D region of interest more accurately (e. g., in case of bad image quality), where the 2D region of interest is determined by the drill tip position in the X-ray. The weighting function may also depend on the C-arm type (flat-panel vs. image intensifier), the type of vertebra (cervical/thoracic/lumbal) and/or the type of 3D data set (pre-operative vs. intra-operative). The system determines a virtual projection of the target point into the X-ray image based on the localization, which projection may be displayed as an overlay in the X-ray image.
7. The system determines the 3D drill tip position in the coordinate system of the 3D data set based on the detected drill tip in the X-ray image by applying the knowledge that both the drill tip and the target point lie on the bone surface. If the distance between the drill tip (i.e., the anchor point) and the target point is below a threshold (e. g., 2 mm; the threshold may depend on the type of vertebra), the system continues with Step 8.
   Otherwise, the system gives an instruction to reposition the drill tip to move it close to the target point. The surgeon follows the instruction and returns to Step 5.
8. In case the drill tip has a remaining distance to the target point, the determination of the 3D drill tip position may take into account a local model of the vertebra's surface in the vicinity of the target point. The local model may be derived from a model of the vertebra in case the vertebra to be drilled has been already classified (i.e., which vertebral level, e.g., L3, L4, etc.).
9. Based on the determined 3D drill tip position in the coordinate system of the 3D data set, the system determines the 3D position and orientation of the drill in the coordinate system of the 3D data set.
10. If the deviation between the 3D drill orientation and the target path is below a threshold (e. g., less than 2 °), continue with Step 12. Otherwise, the system gives an instruction to adjust the drill angle.
11. The surgeon adjusts the drill angle and may return to Step 5 (without the need of changing the imaging direction) or directly continues/commences drilling continues with Step 13. While the surgeon adjusts the drill angle, the system may update the information (e.g., trajectories, positions, angles, etc.) in real time or near real-time.
12. The system calculates the (remaining) drilling depth and gives a corresponding drilling instruction.
13. The surgeon follows the instruction (e. g., by drilling a given distance). During the drilling procedure, the system provides information about the current drilling depth in real time or near real-time. A new X-ray image may be acquired (without the need of changing the imaging direction) whenever a confirmation of the drilling trajectory is desired or advised by the system (e.g., after drilling a certain distance or if drilling close to critical anatomy).
14. The system performs matchings, detections, etc. as described above. Based on the actual drilling start point (this may be, e g., the position of the drill tip as shown in the X-ray image when the first drilling instruction was given), the system determines the 3D position and 3D orientation of the drill.
15. If the remaining drilling distance is below a threshold (e. g., 1 mm), continue with Step 17. If the deviation between the 3D drill orientation and the target path is below a threshold (e. g., 1 °), return to Step 12. Otherwise, the system gives an instruction to adjust the drill angle. If there was a too deep drilling, system may provide a warning.
16. The surgeon follows the instruction, acquires a new X-ray image, and returns to Step 14.
17. The drilling procedure for the current pedicle is completed.
18. For performing the next pedicle drilling return to Step 2.

It is noted again that all references to a "surgeon" in this workflow may refer to a human user as well as a robotic surgeon, a mechanical support device, or a similar apparatus.

In general, particularly in addition to each of the example workflows as disclosed herein, the system may also provide support for performing skin incisions. This may be particularly useful if a surgical robot or a robotic arm is employed. According to an embodiment, a surgical tool (e.g., a scalpel) is aligned with a target trajectory, but with a sufficiently large distance from the target point such that the surgical tool does not yet touch the skin. Using a soft-tissue protection sleeve, the robot or robotic arm may then move the surgical tool along the target trajectory toward the target point until it encounters some resistance (i.e., it touches the skin). Resistance may be detected automatically, e.g., by a pressure sensor. An incision may be made through the soft-tissue protection sleeve. After making the incision, the surgical tool may be exchanged (e.g., switching to a drill while keeping the soft-tissue protection sleeve in place) and the surgical tool may be advanced until it encounters a larger resistance (i.e., it touches the bone surface). Resistance may be detected automatically, e.g., by a pressure sensor. After completing the drilling for one pedicle, the procedure may be repeated for the next pedicle. A pressure sensor may be built into a power tool or a robotic arm holding the surgical object. By recording the pressure that the surgical object exerts on the anatomy, the system may be able to distinguish between, for instance, a drilling process and a slipping movement, where the surgical object slips on an anatomical structure.

It may be helpful if the robot or robotic arm can automatically switch surgical tools (possibly within a soft-tissue protection sleeve), advance or withdraw the soft-tissue protection sleeve, and drill through the soft-tissue protection sleeve (possibly using an oscillating drilling mode). It may be advantageous to specifically design a soft-tissue protection sleeve or adapter suitable for a robot. The design of such a sleeve may also take into account detectability in an X-ray image. For instance, it may be advantageous to make the sleeve from radiotranslucent material or partially from radiotranslucent material (e.g., in the region covering the tip of a drill) such that the surgical object (e.g., the drill and especially its tip) is visible in an X-ray image even when covered by the sleeve. It may also be advantageous to incorporate specific features into the surgical object and/or the sleeve that may be used to make the detection of the surgical object in an X-ray image easier.

A sleeve could also be a patient specific sleeve in case a partial segmentation around the target point is performed preoperatively. The sleeve may e.g. be built by an 3D printer, it may or may not be made of X-ray translucent material. The tip of the patient specific sleeve (the geometry of the opening may be a closed line in 3D space) may fit to the patient's bony anatomy surface around the target point based on a correct rotational alignment of the sleeve. This may have the advantage of a very good fit and may additionally allow for eliminating the need for an accurate navigational information since they support a surgeon (or robot) in finding the desired position of the sleeve tip on the planned target point very accurately. This may also make sure the tip of the sleeve is not positioned in a wrong 3D position, which may occur in some cases where based on a suboptimal imaging directing there are ambiguities of the surface, meaning there are two points on the surface having different 3D coordinates, but appear at the same 2D coordinate in the X-ray image.

A similar approach may also be applied to position an endoscope (the trocar of the endoscope may have a specific extension of its tip) in a desired distance to the surface of the patients bony anatomy, thus is may be possible to use X-ray images from a single viewing direction and still this may allow for positioning the endoscope accurately at the desired 3D position above the bony surface.

Another approach to address the above-described potential ambiguity problem may be to determine the direction of an X-ray beam through the planned or current 2D position of the target point based on the X-ray image and based on the determined viewing direction. After determining the 5DOF position of the region of interest in the X-ray image (i.e. determining a transformation between the X-ray image 3D coordinate system and the data set 3D coordinate system) this beam which is a line in 3D coordinate system of the data set intersects with the 3D data set. In case of 3D dataset that may provide density information (e.g. for a CT), every point (voxel) intersecting with this line shows a density value (e.g. Hounsfield scale). Analyzing all the density values (e.g. analyzing the gradients) of the intersecting voxels of this line may enable to determine whether there is more than one intersection of the line with the bony surface in vicinity (e.g. less than 10 mm) around the target point. In this case, another imaging direction may be needed to avoid the ambiguity and to compute an accurate 3D navigational information. This procedure may be repeated for a plurality of imaging directions to find a suitable imaging direction for a specific target point. During the procedure the system may advice the surgeon or the robotic surgeon to position the imaging device accordingly to prevent inaccurate navigation based on above topological ambiguities.

A sleeve may be X-ray translucent around its tip, and it may not be X-ray translucent further away of its tip (e.g. 40 mm above its tip). In this case this will allow the system to detect the drill bit also inside the sleeve while it may still be possible to determine the 3D position of the sleeve relative to the region of interest.

A fully X-ray translucent sleeve may be tracked e.g. by a real time tracking system and based on a determination of a 3D position of a drill bit based on an X-ray image at a first point in time and knowledge that the drill is inserted in the sleeve at the first point in time. The 3D position of the sleeve relative to the region of interest may be determined at a second point in time. The system may be able to do the same with a sleeve which is partially X-ray translucent and not enough opaque parts of the sleeve have been in the X-ray image to determine its 3D position relative to the region of interest.

According to an embodiment, using a surgical robot may also allow to address the movement between individual vertebrae that are due to the patient breathing. This breathing may be modeled and detected by a pressure sensor. The robot may be capable of keeping the pressure on the vertebra constant when it is not drilling.

This procedure may also be applied for determining the position of a chisel, e.g., during a wedge osteotomy, where the midpoint of a chisel is used as the anchor point.

A similar procedure may also be applicable when using a soft-tissue protection sleeve not made from radiotranslucent material, where the anchor point (e.g., the tip of the drill) may not be visible in the X-ray image. In such a case, instead of using the (not visible) anchor point, a virtual anchor point may be used. This virtual anchor point may be determined based on a previously acquired X-ray image where the anchor point was visible and based on the assumption that there has been no movement of the anchor point between the generation of the current X-ray image and the generation of the previously acquired X-ray image. Based on the current X-ray image, a plane is defined by the axis of the sleeve and the center of the X-ray beam. The virtual anchor point may be determined by orthogonally projecting the anchor point of the previous X-ray image onto that plane. The virtual anchor point may also be determined by choosing the closest point from the anchor point of the previous X-ray image to a contour that lies within the plane (e.g. intersection of the plane with a model of a bone or a partial 3D segmentation of the bone surface) to ensure that the virtual anchor point is positioned on the bone surface. If the anchor point or anchor aspect cannot be seen in any X-ray, it may still be detectable, for example when other prominent points provide sufficient information. For instance, a drill bit may have identifiable flutes that are sufficient to project, where the virtual anchor point is.

If a sleeve is used, then the sleeve itself may be considered a second surgical object that helps with the determination of the 3D spatial relation of the drill bit (the surgical object) relative to the region of interest. The sleeve can be detected in the X-ray image, and the fact that the drill bit is inserted in the drill sleeve (i.e., the axis of the drill sleeve constraints the axis of the drill bit) may be utilized. It may also be possible to track the drill sleeve using an extended reality device (such as head-mounted augmented reality glasses).

In the following we consider this an example for "aligned" surgical tools, where the drill and the sleeve axes are constrained to each other, even though bending or wiggle-room within the sleeve might still need consideration.

Another application of the procedure may be a tumor resection. In this case, a preoperatively acquired MRI scan (e.g., including an outline of a tumor or a planned resection volume) may be fused with a pre- or intraoperatively acquired 3D X-ray scan, where one or more than one target point is identified in the MRI scan and/or the 3D X-ray scan. The surgical instrument may be a resection instrument having an anchor point. By determining the relative 3D position and 3D orientation of the resection instrument and the tumor outline (or planned resection volume), precise 3D navigation instructions may be provided also for the case when several target points have been defined in the planning. Resection may also take place at a certain distance from a target point. Using a robot may also lead to enhanced accuracy. A robot may receive relative 3D positioning information from e.g. internal sensory devices, so the 3D position and 3D orientation of the instrument relative to the resection volume is available at any point in time after the last determination based on acquisition of an X-ray image. A validation of this information is possible at any point in time by acquisition of an additional x-ray image even without using anchor point or target point: Based on the available information from the positioning sensory devices and the last determination of 3D position and 3D orientation of the instrument relative to the resection volume based on the last X-ray image, a virtual X-ray image is generated assuming the imaging direction has not changed. After acquisition of the additional X-ray image from the same imaging direction, the additional X-ray image is compared to the virtual X-ray image and an algorithm decides whether the similarity is high enough (positioning information still valid) or not (there may have been an unknown movement. In latter case a new image with determination of anchor point and target point may be needed.

This procedure may also be combined with a real-time navigation and tracking system. As the procedure is redundant to existing technology and in addition provides a high level of diversification such a combination would make the navigation highly robust and this level of safety may enable autonomous robotic surgery.

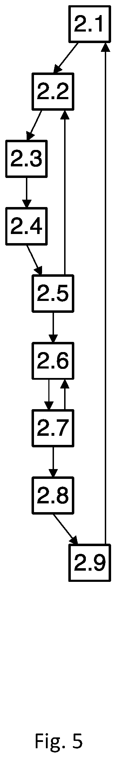

### Example workflow (no planning of target points, single image, but with knowledge about bone surface) for performing a pedicle screw drilling (cf. Fig. 5)

Like in the previous workflows, the numbering of the steps in the workflow of Fig. 5 is a combination of a "2." indicating that the example workflow is the second one out of three, followed by an actual number of the step as used in the following description of the workflow.
1. The surgeon positions the drill tip on the dorsal bone surface in approximate vicinity of the pedicle to be drilled in.
2. The surgeon acquires an X-ray image (e. g., approximately in roughly anterior-posterior (AP) imaging direction), preferably holding the drilling machine in such a way that the surgeon's hand is not in the X-ray beam. If an imaging direction were to lead to the surgeon's hand being in the X-ray beam, the system may provide instructions for a different imaging direction.
3. The system detects the position of the drill tip (i.e., the anchor point) in the X-ray image. The system localizes the region of interest in the X-ray image by computing, from the 3D data set, digitally reconstructed radiographs (DRRs) for a multitude of imaging directions (This may have been performed already before acquisition of the X-ray image). The DRR best matching the X-ray image is taken to localize the area of interest. It is not necessary to restrict the DRR to the region of interest. The system may use a weighting function to match the important regions within the 2D region of interest more accurately (e. g., in case of bad image quality), where the 2D region of interest is determined by the drill tip position in the X-ray. The weighting function may also depend on the C-arm type (flat-panel vs. image intensifier), the type of vertebra (cervical/thoracic/lumbal) and/or the type of 3D data set (pre-operative vs. intra-operative). The system determines a line in the 3D data set defined by the anchor point and the direction of the X-ray beam. The system determines a point on this line where the line intersects with the bone surface (e.g. based on starting at the most posterior point of the line, observing the voxels on this line and determining the voxel with highest gradient of greyscale on this line and possibly in the surrounding of this point). This point defines the position of the anchor point in the 3D data set.
4. Based on the determined 3D drill tip position in the coordinate system of the 3D data set, the system determines the 3D position of the drill in the coordinate system of the 3D data set. The system displays the current position in different views based on the 3D data set, e.g., axial view and sagittal view, it may prolong the trajectory of the drill in order to better visualize the corresponding drilling path. The system may also display detected anatomical structures, like bone cortices, and may also show measurements with respect to those structures or suggest limitations, like cortex near buffer zones that should not be crossed.
5. The surgeon may adjust the drill tip position and/or angle and may return to Step 2 (without the need of changing the imaging direction) or directly commences/continues drilling and continues with Step 6. While the surgeon adjusts the position and/or orientation, the system updates the current position and orientation in the different views (e.g., axial and sagittal) in real time or near time.
6. In order to increase the accuracy, a new X-ray image may be acquired (without the need of changing the imaging direction) whenever a confirmation of the drilling trajectory is desired or (e.g., in case of using a robot for drilling) advised by the system (e.g., after drilling a certain distance or if drilling close to critical anatomy).
7. The system performs matchings, detections, etc. as described above. Based on the actual drilling start point (this may be, e g., the position of the drill tip as shown in the X-ray image when the first drilling instruction was given), the system determines the 3D position of the drill. The system displays the current position of the drill in different views based on the 3D data set, e.g., axial view and sagittal view. If the surgeon does not accept the current position as being ok, he/she may correct the angulation (e.g., while drill bit is running) and proceeds to step 6.
8. The system provides information about the current drilling depth in real time or near time. Based on this information the surgeon decides if the drilling procedure for the current pedicle is completed.
9. For performing the next pedicle drilling return to Step 1.

Also here, all references to a "surgeon" in this workflow may refer to a human user as well as a robotic surgeon, a mechanical support device, or a similar apparatus.

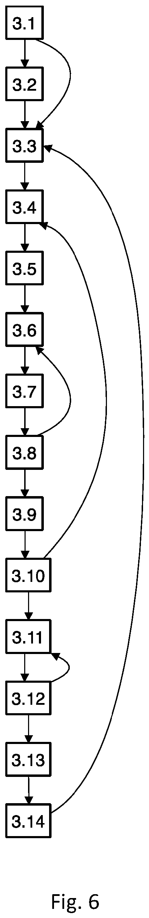

### Example workflow (no planning of target points, dual image) for performing a pedicle screw drilling (cf. Fig. 6)

Like in the previous workflows, the numbering of the steps in the workflow of Fig. 6 is a combination of a "3." indicating that the example workflow is the third one out of three, followed by an actual number of the step as used in the following description of the workflow.
1. If the system utilizes external real time (or near real time) tracking, it may continuously track objects once available. Objects that could be used are the drill bit, the drilling machine or the sleeve, to at least partially determine the drill position independently of the X-rays. If a tracking system is not available, it may continue at Step 3.
2. Additionally, the system may continuously detect a second reference (e.g. anatomy in region of interest or tracking of the sleeve positioned on bony surface of the anatomy in the region of interest) and/or model the movement of the region of interest to improve the tracking accuracy of the drill bit relative to the region of interest in order to allow for distinguishing between e.g. breathing movement of the patient and entry depth of a drill as the sleeve tip may always stay on the bone surface independent of breathing movements.
3. The surgeon positions the drill tip on the dorsal bone surface in approximate vicinity of the pedicle to be drilled in. The surgeon starts drilling a short distance (e.g., 3 mm) in the approximate needed direction.
4. The surgeon acquires an X-ray image (e. g., approximately in roughly anterior-posterior (AP) imaging direction), preferably holding the drilling machine in such a way that the surgeon's hand is not in the X-ray beam. If an imaging direction were to lead to the surgeon's hand being in the X-ray beam, the system may provide instructions for a different imaging direction.
5. The system detects the position of the drill tip (i.e., the anchor point) in the X-ray image. The system localizes the region of interest in the X-ray image by computing, from the 3D data set, digitally reconstructed radiographs (DRRs) for a multitude of imaging directions (This may have been performed already before acquisition of the X-ray image). The DRR best matching the X-ray image is taken to localize the area of interest. It is not necessary to restrict the DRR to the region of interest. The system may use a weighting function to match the important regions within the 2D region of interest more accurately (e. g., in case of bad image quality), where the 2D region of interest is determined by the drill tip position in the X-ray. The weighting function may also depend on the C-arm type (flat-panel vs. image intensifier), the type of vertebra (cervical/thoracic/lumbal) and/or the type of 3D data set (pre-operative vs. intra-operative).
6. The surgeon acquires a further X-ray image. The drill tilt may change between the acquisition of the image from Step 4 and this image, but it needs to be ensured that the drill tip remains in place, which may be easy as it is already inside the bone (see Step 3). Alternatively, the surgeon could have drilled between the first and the further X-ray image while the drill axis in the further X-ray remains close to the anchor point (where the tip was placed) of the first X-ray.
7. The system detects the position of the drill tip (i.e., the anchor point) in the further X-ray image and localizes the region of interest in the further X-ray image.
8. The system registers both X-ray images based on the localizations of the region of interest. Based on this image registration and the detected drill tips, the system calculates the epipolar lines for the detected drill tips and determines the 3D drill tip position in the coordinate system of the 3D data set by calculating the closest point between the epipolar lines. In case the surgeon drilled between the first and the further image, the epipolar lines will be further apart. However, the axis of the drill in the further image represents a plane in the 3D coordinate system, whose intersection with the epipolar line of the drill tip in the first X-ray provides the 3D drill tip position in the first X-ray image. This is accurate enough, when the viewing directions onto the drill have sufficiently changed, e.g. between 10 and 120 degrees between the first and the further image. If successful, continue with Step 9, otherwise continue with Step 6.
9. Based on the determined 3D drill tip position (from at least the first image) in the coordinate system of the 3D data set, the system determines the 3D position and orientation of the drill in the coordinate system of the 3D data set (in both images). The system displays the current position in different views based on the 3D data set, e.g., axial view and sagittal view, it may prolong the trajectory of the drill in order to better visualize the corresponding drilling path.
10. The surgeon may adjust the drill tip position and/or angle and may return to Step 4 (without the need of changing the imaging direction) or directly commences/continues drilling and continues with Step 11. While the surgeon adjusts the position and/or orientation, the system updates the current position and orientation in the different views (e.g., axial and sagittal) in real time or near-real time.
11. In order to increase the accuracy, a new X-ray image may be acquired (without the need of changing the imaging direction) whenever a confirmation of the drilling trajectory is desired or (e.g. in case of using a robot for drilling) advised by the system (e.g., after drilling a certain distance or if drilling close to critical anatomy).
12. The system performs matchings, detections, etc. as described above. Based on the actual drilling start point (this may be, e g., the position of the drill tip as shown in the X-ray image when the first drilling instruction was given), the system determines the 3D position and 3D orientation of the drill. The system displays the current position of the drill in different views based on the 3D data set, e.g. axial view and sagittal view. If the surgeon does not accept the current position as being ok, he/she may correct the angulation (e.g. while drill bit is running) and proceeds to Step 11.
13. The system provides information about the current drilling depth in real time or near time. Based on this information the surgeon decides if the drilling procedure for the current pedicle is completed.
14. For performing the next pedicle drilling return to Step 3.

While embodiments have been illustrated and described in detail in the drawings and aforegoing description, such illustrations and descriptions are to be considered illustrative or exemplary and not restrictive, and the invention is not limited to the disclosed embodiments.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims.

The mere fact that certain measures are recited in mutually different dependent claims does not indicate that a combination of these measures cannot be used to advantage.

## Claims

1. A system for determining a 3D position of a surgical object relative to a region of interest in a patient's anatomy, the system comprising a processing unit and a computer program product including instructions which, when executed on the processing unit of the system, cause the system
to receive a first X-ray image generated with a first imaging direction at a first point in time, wherein the X-ray image is a 2D projection image defining a first 3D image coordinate system, the 2D projection image depicting part of the patient's anatomy and at least part of the surgical object, the surgical object having a geometrical aspect,
to receive a 3D data set describing the part of the patient's anatomy and the region of interest in a 3D data coordinate system,
to localize the part of the patient's anatomy based on the first X-ray image in order to determine a spatial relation between the first 3D image coordinate system and the 3D data coordinate system,
to determine the 3D position of the surgical object relative to the region of interest in the 3D data coordinate system at the first point in time based on
(I) the determined spatial relation between the first 3D image coordinate system and the 3D data coordinate system,
(II) a spatial relation between the surgical object and the first 3D image coordinate system, and
(III) information related to the geometrical aspect,
wherein the spatial relation between the surgical object and the first 3D image coordinate system is derived by at least one out of the group of
(A) inferring a neural network,
(B) utilizing a plurality of virtually generated X-ray images, with the images depicting parts of the surgical object from different imaging directions, and
(C) applying an algorithm utilizing a 3D model representing at least a part of the surgical object,
wherein the information related to the geometrical aspect includes at least one information out of the group of
(a) the geometrical aspect of the surgical object is positioned near a surface of at least part of the region of interest,
(b) a point, an axis or a plane in the first 3D image coordinate system which constraints the possible options for spatial relation of the geometrical aspect to the first 3D image coordinate system,
(c) a 3D position of a target point in the 3D data coordinate system, which is near to the geometrical aspect, with the projections in the 2D projection image of the target point and the projection of the geometrical aspect in the 2D projection image being near to each other.

2. The system according to claim 1, wherein the computer program product including instructions which, when executed on the processing unit of the system, cause the system
to receive a second X-ray image generated with a second imaging direction, with the position of the geometrical aspect of the surgical object being similar in the 3D data coordinate system, and
to localize the part of the anatomy based on the second X-ray image in order to determine a spatial relation between the second 3D image coordinate system and the 3D data coordinate system,
wherein determining the 3D position of the surgical object relative to the region of interest in the 3D data coordinate system at the first point in time is further based on
(IV) the determined spatial relation between the second 3D image coordinate system and the 3D data coordinate system, and
(V) a spatial relation between the geometrical aspect of the surgical object and the second 3D image coordinate system,
wherein the spatial relation between the geometrical aspect of the surgical object and the second 3D image coordinate system is derived by at least one out of the group of
(A) inferring a neural network,
(B) utilizing a plurality of virtually generated X-ray images, with the images depicting parts of the surgical object from different imaging directions, and
(C) applying an algorithm utilizing a 3D model representing at least a part of the surgical object.

3. The system according to any one of claims 1 and 2, wherein the 3D data set is updated based on at least the first X-ray image and/or the second X-ray image.

4. The system of any one of claims 1 to 3, wherein the surgical object is a drill bit and the region of interest in the patient's anatomy is a bone structure.

5. The system of any one of claims 1 to 3, wherein the surgical object has a main axis and wherein the first X-ray image and/or the second X-ray image depict at least part of the surgical object together with at least part of a further surgical object having a main axis and a tip-point, with the main axis of the surgical object and the main axis of the further surgical object being aligned with each other in 3D space, wherein the further surgical object is a drill bit,
wherein the computer program product includes instructions which, when executed on the processing unit of the system, cause the system
to determine the 3D position of the further surgical object relative to the region of interest in the 3D data coordinate system based on the spatial relation of the surgical object relative to the region of interest and on at least one out of the group of
(i) the position of a tip-point of the further surgical object in the first and/or second X-ray image,
(ii) a physical scale at the further surgical object showing an entry depth of the further surgical object; and
(iii) an entry depth provided by internal sensors of a robot holding the further surgical object.

6. The system according to claim 1, wherein the surgical object has a deformation such that a tip-point deviates from a main axis of the surgical object, wherein the computer program product includes instructions which, when executed on the processing unit of the system, cause the system
to detect the deformation of the surgical object by fusing an updated position with the determined 3D position of the surgical object, in order to determine one or more of the group of
(i) a 3D position of the tip-point in the 3D data coordinate system, and
(ii) the geometrical aspect of the surgical object in the 3D data coordinate system, such as the projected trajectory of the tip-point of the surgical object.

7. The system of any one of claims 1 to 4, wherein the computer program product includes instructions which, when executed on the processing unit of the system, cause the system
to determine a 3D position of the surgical object relative to the region of interest at a second point in time based on
(i) the 3D position of the surgical object relative to the region of interest at the first point in time, and
(ii) information about a change of 3D position of the surgical object between the first point in time and the second point in time, the information being provided by a tracking based on at least one out of the group of a 3D sensor; planned movement of a robotic arm; a plurality of 2D cameras; a head mounted augmented reality device.

8. The system of claim 5, wherein the computer program product includes instructions which, when executed on the processing unit of the system, cause the system
to determine an insertion depth of the further surgical object in the region of interest at a second point in time based on at least one out of the group of
(i) tracking by a camera-based and/or sensor-based tracking of a tool attached to the further surgical object,
(ii) knowledge that a tip-point of the further surgical object was positioned at the surface of the region of interest at the first point in time,
(iii) knowledge of an insertion depth of the further surgical object at the first point in time,
(iv) a 3D position of the further surgical object in the 3D data coordinate system at the second point in time,
(v) knowledge about the distance between the tip-point of the further surgical object and the geometrical aspect of the surgical object.

9. The system of claim 5, wherein the computer program product includes instructions which, when executed on the processing unit of the system, cause the system
to determine a 3D position of the further surgical object relative to the region of interest at the second point in time based on
(i) the insertion depth of the further surgical object at the second point in time, and
(ii) the 3D position of the surgical object relative to the part of the region of interest at the second point in time.

10. The system of claim 1, wherein a robot holds the surgical object and determines an insertion depth into the region of interest based on internal sensory data of the robot between the first point in time and the second point in time.

11. The system of any one of claims 1 to 10, further comprising a robot, wherein the robot is configured to control a position and a change of the position of at least one out of the group of an imaging device, the surgical object, the further surgical object, the region of interest.

12. A method for determining a 3D position of a surgical object relative to a region of interest in a patient's anatomy, the method comprising
receiving a first X-ray image generated with a first imaging direction at a first point in time, wherein the X-ray image is a 2D projection image defining a first 3D image coordinate system, the 2D projection image depicting part of the patient's anatomy and at least part of the surgical object, the surgical object having a geometrical aspect,
receiving a 3D data set describing the part of the patient's anatomy and the region of interest in a 3D data coordinate system,
localizing the region of interest based on the first X-ray image,
determining a spatial relation between the first 3D image coordinate system and the 3D data coordinate system based on the localized region of interest,
determining the 3D position of the surgical object relative to the region of interest in the 3D data coordinate system based on
(I) the determined spatial relation between the first 3D image coordinate system and the 3D data coordinate system,
(II) a spatial relation between the surgical object and the first 3D image coordinate system, and
(III) information related to the geometrical aspect,
wherein the spatial relation between the surgical object and the first 3D image coordinate system is derived by at least one out of the group of
(A) inferring a neural network,
(B) utilizing a plurality of virtually generated x-ray images, with the images depicting different parts of the surgical object from different imaging directions, and
(C) applying an algorithm utilizing a 3D model representing at least a part of the surgical object,
wherein the information related to the geometrical aspect includes at least one information out of the group of
(a) the geometrical aspect of the surgical object is positioned near a surface of at least part of the region of interest,
(b) a point or axis in the first 3D image coordinate system which constraints the possible options for spatial relation of the geometrical aspect,
(c) a 3D position of a target point in the 3D data coordinate system, which is near to the geometrical aspect, with the projections in the 2D projection image of the target point and the geometrical aspect being near to each other.

13. A computer program product including instructions, when executed on a processing unit of the system according to claim 1, causing the system to perform the method according to claim 12.
